(19) Europäisches Patentamt
European Patent Office
Office européen des brevets

(11) Numéro de publication : **0 368 763 B1**

# (12) FASCICULE DE BREVET EUROPEEN

(45) Date de publication du fascicule du brevet : **14.10.92 Bulletin 92/42**

(51) Int. Cl.$^5$ : **C07C 323/52,** A61K 7/09, C07D 295/185

(21) Numéro de dépôt : **89403099.8**

(22) Date de dépôt : **09.11.89**

(54) **Mercapto-4 butyramides N-mono ou N,N-disubstitués par un radical mono ou polyhydroxyalkyle, et leur utilisation en tant qu'agents réducteurs dans un procédé de déformation permanente des cheveux.**

(30) Priorité : **09.11.88 LU 87378**

(43) Date de publication de la demande : **16.05.90 Bulletin 90/20**

(45) Mention de la délivrance du brevet : **14.10.92 Bulletin 92/42**

(84) Etats contractants désignés : **AT BE CH DE ES FR GB GR IT LI NL SE**

(56) Documents cités :
**AT-B- 348 546**
**CH-A- 511 604**
**PATENT ABSTRACTS OF JAPAN, vol. 11, no. 115 (C-415)[2562], 10 avril 1987; & JP-A-61 260 058**

(73) Titulaire : **L'OREAL**
**14, Rue Royale**
**F-75008 Paris (FR)**

(72) Inventeur : **Maignan, Jean**
**8, Rue Halévy**
**F-93290 Tremblay les Gonesse (FR)**
Inventeur : **Colin, Michel**
**10, Allée Cécile**
**F-93190 Livry Gargan (FR)**
Inventeur : **Lang, Gérard**
**44, Avenue Lacour**
**F-95210 Saint Gratien (FR)**

(74) Mandataire : **Stalla-Bourdillon, Bernard et al**
**CABINET NONY & CIE 29, rue Cambacérès**
**F-75008 Paris (FR)**

EP 0 368 763 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux mercapto-4 butyramides N-mono ou N,N-disubstitués par un radical mono ou polyhydroxyalkyle et leur utilisation, en tant qu'agents réducteurs, dans un procédé de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou décrépage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un mercaptan.

Parmi ces derniers, ceux couramment utilisés sont l'acide thioglycolique et l'acide thiolactique ou un mélange de ces acides ainsi que leurs esters par exemple le monothioglycolate de glycérol ou de glycol.

Ces agents réducteurs sont particulièrement efficaces pour réduire les liaisons disulfures de la kératine notamment l'acide thioglycolique qui peut être considéré comme le produit de référence en permanente, et qui conduit à un taux de réduction d'environ 50%.

Ces agents réducteurs présentent cependant un inconvénient majeur dans la mesure où ils dégagent de mauvaises odeurs, d'ailleurs propres aux composés soufrés qui rendent les opérations de permanente parfois pénibles non seulement pour les personnes qui les subissent mais également pour les personnes qui les effectuent.

En vue de pallier à cet inconvénient, il est généralement fait usage d'un parfum permettant de masquer les odeurs.

Des études ont par ailleurs été conduites en vue de mettre au point de nouveaux agents réducteurs sans odeur. Ainsi, dans la demande de brevet japonais (KOKAI) n°260.058/86, il a été proposé l'utilisation de N-mercaptoalkylgluconamides qui sont des composés solubles dans l'eau, de faible volatilité et pratiquement dénués d'odeur.

Ces N-mercaptoalkylgluconamides sont obtenus par réaction de la glucono-$\delta$-lactone avec un aminoalcanethiol.

Ces nouveaux agents réducteurs, s'ils permettent de remédier aux inconvénients des agents réducteurs connus, ne possèdent pas toutefois d'excellentes propriétés réductrices par rapport notamment à l'acide thioglycolique.

Des amides de l'acide thioglycolique et de l'acide mercapto-3 propionique sont également connus (voir J.W. HAEFELE et coll, Proceedings of Scientific Section, 1959, 32 p.52) et ont été étudiés en sensibilisation cutanée (voir J. Invest. Dermatol. 32, 273-279, 1958). Selon le test employé pour cette étude le N-hydroxyéthyl thioglycolamide et le N-(hydroxyéthyl) mercapto-3 propionamide se sont avérés être sensibilisants.

On a maintenant constaté, de façon tout à fait surprenante qu'en utilisant une nouvelle classe de mercaptoalkylamides, à savoir des mercapto-4 butyramides N-mono ou N,N-disubstitués par un radical mono ou polyhydroxyalkyle, il était possible de remédier aux inconvénients des agents réducteurs mentionnés ci-dessus.

Les mercapto-4 butyramides selon l'invention sont d'une façon générale moins toxiques et conviennent donc tout particulièrement, du fait de leurs excellentes propriétés réductrices, comme agents réducteurs dans les procédés de déformation permanente des cheveux.

Les propriétés réductrices sont en effet tout à fait comparables à celles de l'acide thioglycolique mais l'odeur, qui est moins prononcée et d'ailleurs différente de celle de l'acide thioglycolique, est beaucoup plus facile à masquer lors de l'utilisation.

La présente invention a pour objet, à titre de produits industriels nouveaux, des mercapto-4 butyramides N-mono ou N,N-disubstitués par un radical mono ou polyhydroxyalkyle, solubles dans l'eau, correspondant à la formule générale suivante:

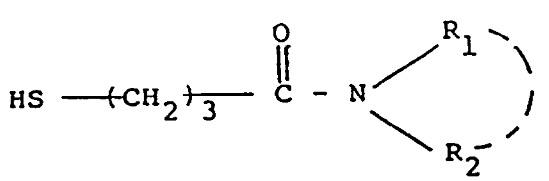

(I)

dans laquelle:

$R_1$ représente un atome d'hydrogène, un radical alkyle inférieur ou un radical monohydroxyalkyle ou polyhydroxyalkyle ayant de 1 à 6 atomes de carbone, éventuellement interrompus par un atome d'oxygène, d'azote ou de soufre,

et $R_2$ représente un radical monohydroxyalkyle ou polyhydroxyalkyle ayant de 1 à 6 atomes de carbone éventuellement interrompus par un atome d'oxygène, d'azote ou de soufre,

ou $R_1$ et $R_2$, pris ensemble avec l'atome d'azote, forment un cycle pipérazine substitué en 4 par un radical mono ou polyhydroxyalkyle ayant de 1 à 6 atomes de carbone.

Par radical alkyle inférieur on doit entendre un radical ayant de 1 à 6 atomes de carbone et de préférence le radical méthyle, éthyle, propyle, isopropyle et butyle.

Parmi les radicaux monohydroxyalkyle et polyhydroxyalkyles ayant de 1 à 6 atomes de carbone, éventuellement interrompus par un atome d'oxygène, d'azote ou de soufre on peut mentionner: le radical hydroxy-2 éthyle, dihydroxy-2,3 propyle, diméthyl-1,1 hydroxy-2 éthyle, pentahydroxy-2,3,4,5,6 hexyle (reste du sorbitol) et hydroxy-2 éthyloxyéthyle.

Parmi les mercapto-4 butyramides de formule (I) ci-dessus on peut notamment citer les suivants:

- le N-(hydroxy-2 éthyl) mercapto-4 butyramide,
- le N-(hydroxy-2 éthyloxyéthyl) mercapto-4 butyramide,
- le N-(dihydroxy-2,3 propyl) mercapto-4 butyramide,
- le N,N-di(hydroxy-2 éthyl) mercapto-4 butyramide,
- la N-(mercapto-4 butyryl) N-méthylglucamine,
- la N-(mercapto-4 butyryl-1) N'-(hydroxy-2 éthyl) pipérazine,
- le N-(diméthyl-1,1 hydroxy-2 éthyl) mercapto-4 butyramide.

La présente invention a également pour objet le procédé de préparation des mercapto-4 butyramides selon l'invention, ce procédé consistant à faire réagir une amine (1) sur la γ-thiobutyrolactone (2) selon le schéma réactionnel suivant:

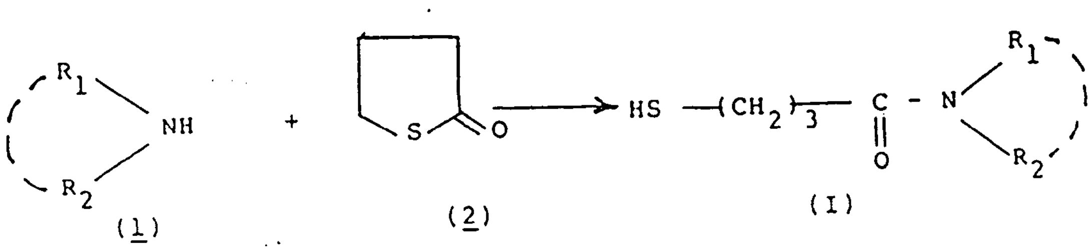

(1) (2) (I)

Parmi les amines (1) on peut notamment mentionner: l'éthanolamine, la diéthanolamine, la diglycolamine, la méglumine ou N-méthylglucamine, la dihydroxy-2,3 propylamine, l'amino-2 méthyl-2 propanol et l'hydroxy-2 éthyl pipérazine.

La γ-thiobutyrolactone (2) est un produit commercial.

La réaction d'ouverture de la γ-thiobutyrolactone est généralement conduite sous atmosphère inerte dans un solvant aromatique tel que le toluène ou un alcool tel que le méthanol, l'éthanol, l'isopropanol ou le butanol, et, suivant le point d'ébullition du solvant, à une température comprise entre 50 et 110°C.

Toutefois selon une forme préférée de l'invention la réaction est conduite sans solvant et dans ce cas on mélange dans des proportions stoechiométriques l'amine (1) et la γ-thiobutyrolactone (2), puis on porte le mélange, sous atmosphère inerte, à une température comprise entre 50 et 110°C.

Suivant la basicité de l'amine (1), le temps de réaction peut être compris entre 2 et 8 heures, l'évolution de la réaction étant suivie en dosant l'apparition du thiol et la disparition de la fonction amine.

Si au cours de la réaction une certaine quantité de thiol est oxydée en disulfure correspondant, le mélange réactionnel est alors dilué par deux fois son volume d'eau et agité en présence d'un mélange de résine sulfonique et de poudre de zinc pendant 3 à 10 heures.

La majorité du disulfure étant réduite, le mélange est alors filtré et on obtient une solution du composé attendu qui peut être utilisée directement.

Ce traitement à l'aide d'une résine sulfonique permet par ailleurs de fixer les traces d'amine libre (1) n'ayant pas réagi.

Les mercapto-4 butyramides peuvent également être obtenus par réaction d'amidification de l'acide mercapto-4 butyrique ou de ses esters. Cependant le procédé de préparation décrit ci-dessus à partir de la γ-thiobutyrolactone est plus rapide et n'entraîne pas la formation de produits secondaires qui parfois sont très malodorants et contaminent le produit final.

La présente invention a également pour objet à titre de produits industriels nouveaux les disulfures des composés de formule (I) ceux-ci pouvant être représentés par la formule générale suivante :

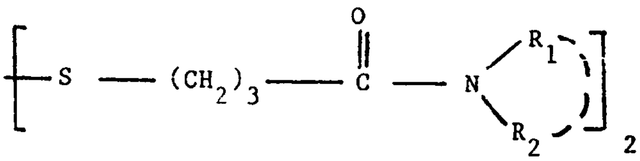 (II)

dans laquelle :

$R_1$ et $R_2$ ont les mêmes significations que celles données ci-dessus pour la formule (I).

Les disulfures trouvent une application dans les compositions dites "auto-neutralisantes" c'est-à-dire lorsqu'ils sont associés à un thiol, tel que par exemple un composé de formule (I) ci-dessus, dans des proportions molaires allant de 0,5 à 2,5 et de préférence de 1 à 2 (voir Brevet US 3.768.490).

Les disulfures de formule (II) selon l'invention sont obtenus par oxydation des composés de formule (I) soit à l'air, soit en utilisant par exemple de l'eau oxygénée en présence éventuellement d'ions ferreux.

Parmi les disulfures de formule (II) ci-dessus on peut notamment citer les suivants :

le bis[N-(dihydroxy-2,3 propyl butyramide)]disulfure,

le bis[N-(hydroxy-2 ethyloxyéthyl butyramide)]disulfure,

le bis [N-(butyryl-1) N'-(hydroxy-2 éthyl) pipérazine] disulfure.

La présente invention a également pour objet une composition réductrice, pour le premier temps d'une opération de déformation permanente des cheveux, contenant dans un véhicule cosmétique approprié, au moins un mercapto-4 butyramide de formule (I) telle que définie ci-dessus, en tant qu'agent réducteur.

De préférence, l'agent réducteur est présent dans la composition selon l'invention à une concentration comprise entre 2 et 20% en poids et de préférence entre 5 et 10% en poids par rapport au poids total de la composition réductrice.

A la composition réductrice selon l'invention, on pourra ajouter d'autres réducteurs et, en particulier, le thioglycérol, l'acide thioglycolique et ses esters, l'acide thiolactique et ses esters, la cytéamine et ses dérivés ainsi que les disulfures correspondants tels que la cystamine, la cystine, l'acide dithioglycolique, etc.

Le pH de la composition est généralement compris entre 4 et 11, et obtenu à l'aide d'agents alcalins tels que par exemple l'ammoniaque, la monoéthanolamine, la diéthanolamine, la triéthanolamine, un carbonate ou un bicarbonate alcalin ou d'ammonium.

La composition réductrice peut également contenir divers ingrédients tels que par exemple des polymères cationiques tels que ceux utilisés dans les compositions des brevets français n°79.32078 et 80.26421 ou encore des polymères cationiques du type ionène tels que ceux utilisés dans les compositions du brevet français n°82.17364, des agents adoucissants et notamment des ammoniums quaternaires dérivés de la lanoline, des hydrolysats de protéines, des cires, des agents opacifiants, des parfums, des colorants, des tensio-actifs ou encore des agents traitants.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrépage des cheveux, la composition réductrice est de préférence sous forme d'une crème de façon à maintenir les cheveux aussi raides que possible. On réalise ces crèmes, sous forme d'émulsions, par exemple à base de stéarate de glycéryle, de stéarate de glycol, de cires auto-émulsionnables, d'alcools gras etc... On peut également utiliser des liquides ou des gels contenant des agents épaississants tels que des polymères ou copolymères carboxyvinyliques qui "collent" les cheveux et les maintiennent dans la position lisse pendant le temps de pose.

La présente invention a également pour objet un procédé de déformation permanente des cheveux consistant, dans une première étape, à réduire les liaisons disulfures de la kératine par application, pendant environ 5 à 60min, d'une composition réductrice telle que définie ci-dessus puis dans une seconde étape à reformer lesdites liaisons par application d'une composition oxydante ou éventuellement en laissant agir l'oxygène de l'air.

Lorsqu'il s'agit d'un procédé d'ondulation des cheveux, on applique une composition réductrice telle que définie ci-dessus sur des cheveux mouillés préalablement roulés sur des rouleaux ayant de 4 à 20mm de diamètre, la composition pouvant éventuellement être appliquée au fur et à mesure de l'enroulage des cheveux;

on laisse ensuite agir la composition réductrice pendant un temps de 5 à 60min, de préférence de 5 à 30min puis on rince abondamment après quoi on applique, sur les cheveux enroulés, une composition oxydante permettant de reformer les liaisons disulfures de la kératine pendant un temps de pose de 2 à 10min. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel ou un mélange de bromate alcalin et d'un persel. Cette oxydation peut-être immédiate ou différée. La concentration en eau oxygénée peut varier de 1 à 10 volumes, la concentration en bromate alcalin de 2 à 12% et celle en persel de 0,1 à 15% en poids par rapport au poids total de la composition oxydante.

Lorsqu'il s'agit d'un procédé de défrisage ou de décrépage des cheveux, on applique sur les cheveux une composition réductrice selon l'invention puis l'on soumet les cheveux à une déformation mécanique permettant de les fixer dans leur nouvelle forme, par une opération de lissage des cheveux avec un peigne à large dents, avec le dos d'un peigne ou à la main. Après un temps de pose de 5 à 60min, en particulier de 5 à 30min, on procède alors à un nouveau lissage puis on rince soigneusement et on applique la composition oxydante ou fixatrice que l'on laisse agir pendant 2 à 10min environ puis on rince abondamment les cheveux.

On va maintenant donner à titre d'illustration et sans aucun caractère limitatif plusieurs exemples de préparation des mercapto-4 butyramides ainsi que plusieurs exemples de compositions réductrices les contenant.

EXEMPLE I

Préparation du N-(hydroxy-2 éthyl) mercapto-4 butyramide

A 61,10g d'éthanolamine agitée sous atmosphère inerte a 75°C on ajoute, en environ 15mn, 102g de γ-thiobutyrolactone. La température s'élève jusqu'à 90°C au cours de l'addition et à la fin de celle-ci le mélange est encore agité pendant une heure à une température comprise entre 85 et 90°C, temps au bout duquel l'éthanolamine est totalement transformée.

On obtient 160g de N-(hydroxy-2 éthyl) mercapto-4 butyramide sous forme liquide à température ordinaire dont le dosage en thiol et le spectre de $^1H$ R.M.N. sont conformes à la structure attendue.

L'analyse élémentaire correspond à un produit partiellement hydraté: $C_6H_{13}NO_2S$, 0,25 $H_2O$.

| | C | H | N | O | S |
|---|---|---|---|---|---|
| Calc: | 41,80 | 8,19 | 8,13 | 23,22 | 18,61 |
| Tr: | 42,09 | 7,89 | 8,15 | 23,20 | 18,70 |

EXEMPLE II

Préparation du N-(hydroxy-2 éthyloxyéthyl) mercapto-4 butyramide

En une demi-heure, on ajoute 102g de γ-thiobutyrolactone, goutte à goutte, à 105g de diglycolamine agitée sous atmosphère inerte à 70°C. La température s'élève jusqu'à 95°C au cours de l'addition. Celle-ci étant terminée, le mélange est encore agité pendant 2 heures à 85°C, temps au bout duquel toute l'amine de départ est transformée. On obtient 205g de N-(hydroxy-2 éthyloxyéthyl) mercapto-4 butyramide sous forme d'un liquide légèrement rose. Le dosage de thiol et le spectre $^1H$ R.M.N. correspondent à la structure attendue.

L'analyse élémentaire correspond à un produit partiellement hydraté: $C_8H_{17}NO_3S$, 0,25 $H_2O$.

| | C | H | N | O | S |
|---|---|---|---|---|---|
| Calc: | 45,36 | 8,32 | 6,61 | 24,55 | 15,14 |
| Tr: | 45,66 | 8,27 | 6,70 | 24,29 | 15,28 |

EXEMPLE III

Préparation du N-(dihydroxy-2,3 propyl) mercapto-4 butyramide

A 91,1g de dihydroxy-2,3 propylamine agitée sous atmosphère inerte à une température de 80°C on ajoute,

goutte à goutte, 102g de γ-thiobutyrolactone. La réaction est exothermique. L'addition terminée, le mélange est encore agité pendant 2 heures à une température comprise entre 80° et 90°C temps au bout duquel toute l'amine est transformée. On obtient 190g de N-(dihydroxy-2,3 propyl) mercapto-4 butyramide sous forme d'un solide blanc dont le point de fusion est de : 48°C. Le dosage de thiol et le spectre $^1H$ R.M.N. correspondent à la structure attendue.

L'analyse élémentaire correspond à un produit partiellement hydraté: $C_7H_{15}NO_3S$, 0,25 $H_2O$.

| | C | H | N | O | S |
|---|---|---|---|---|---|
| Calc: | 42,51 | 7,90 | 7,08 | 26,29 | 16,21 |
| Tr: | 42,76 | 7,89 | 7,10 | 26,09 | 16,40 |

EXEMPLE IV

Préparation de la N-(mercapto-4 butyryl-1) N'-(hydroxy-2 éthyl) pipérazine

A 130,2g de N-(hydroxy-2 éthyl) pipérazine agitée sous atmosphère inerte à une température de 60°C on ajoute en une demi-heure 102g de γ-thiobutyrolactone. Ensuite le mélange est maintenu pendant 2 heures à une température d'environ 90-100°C temps au bout duquel toute la γ-thiobutyrolactone est transformée. On obtient 230g de N-(mercapto-4 butyryl-1) N'-(hydroxy-2 éthyl) pipérazine sous forme d'un liquide visqueux dont le dosage en thiol et le spectre $^1H$ R.M.N. correspondent à la structure attendue.

L'analyse élémentaire correspond à un produit partiellement hydraté: $C_{10}H_{20}N_2O_2S$; 0,5 $H_2O$.

| | C | H | N | O | S |
|---|---|---|---|---|---|
| Calc: | 49,76 | 8,77 | 11,60 | 16,57 | 13,28 |
| Tr: | 49,37 | 8,72 | 11,39 | 17,13 | 13,47 |

EXEMPLE V

Préparation du N,N-di(hydroxy-2 éthyl) mercapto-4 butyramide

A 105,14g de diéthanolamine agitée sous atmosphère inerte à 70-80°C on ajoute, goutte à goutte, 102g de γ-thiobutyrolactone.

On ne constate aucune exothermicité et le mélange est agité pendant 6 heures à une température comprise entre 90° et 100°C.

On obtient 206g de N,N-di(hydroxy-2 éthyl) mercapto-4 butyramide sous forme d'un liquide visqueux à température ordinaire. Les dosages de thiol, d'amine résiduelle et le spectre $^1H$ R.M.N. correspondent à la structure attendue.

L'analyse élémentaire correspond à un produit partiellement hydraté: $C_8H_{17}NO_3S$; 0,25 $H_2O$

| | C | H | N | O | S |
|---|---|---|---|---|---|
| Calc: | 45,36 | 8,32 | 6,61 | 24,55 | 15,13 |
| Tr: | 45,13 | 8,00 | 6,47 | 25,53 | 15,05 |

EXEMPLE VI

Préparation de la N-(mercapto-4 butyryl) N-méthylglucamine

Un mélange de 102g de γ-thiobutyrolactone et de 195,2g de N-méthylglucamine est porté progressivement sous atmosphère inerte à une température comprise entre 90 et 100°C. Ce mélange est agité à cette température pendant 6 heures, temps au bout duquel la réaction est terminée.

On obtient 296g de N-(mercapto-4 butyryl) N-méthylglucamine sous forme d'un solide vitreux dont le dosage en thiol et le spectre $^1H$ R.M.N. correspondent à la structure attendue.

L'analyse élémentaire correspond à un produit partiellement hydraté: $C_{11}H_{23}NO_6S$; 0,75 $H_2O$.

| | C | H | N | O | S |
|---|---|---|---|---|---|
| Calc: | 42,49 | 7,94 | 4,50 | 34,73 | 10,31 |
| Tr: | 42,89 | 7,57 | 4,61 | 34,84 | 10,42 |

EXEMPLE VII

Préparation du N-(diméthyl-1,1 hydroxy-2 éthyl) mercapto-4 butyramide

En une demi-heure, on ajoute 102g de γ-thiobutyrolactone, goutte à goutte, à 89,14g d'amino-2 méthyl-1 propanol agité sous atmosphère inerte à 65°C. La température s'élève à 70°C au cours de l'addition. Celle-ci étant terminée, le mélange est encore agité pendant 8 heures à 100°C, temps au bout duquel toute l'amine de départ est transformée.

On obtient 180g de N-(diméthyl-1,1 hydroxy-2 éthyl) mercapto-4 butyramide sous forme d'un liquide visqueux.

Le spectre $^1H$ R.M.N. correspond à la structure attendue et l'analyse élémentaire à un produit partiellement hydraté: $C_8H_{17}NO_2S$, 0,25 $H_2O$.

| | C | H | N | O | S |
|---|---|---|---|---|---|
| Calc: | 49,07 | 8,94 | 7,15 | 18,39 | 16,37 |
| Tr: | 48,87 | 8,58 | 6,36 | 17,89 | 17,40 |

EXEMPLE VIII

Préparation du bis[N-(dihydroxy-2,3 propyl butyramide)] disulfure

A une solution de 15g (0,077 mole) de N-(dihydroxy-2,3 propyl) mercapto-4 butyramide dans 75 $cm^3$ d'eau agitée à température ambiante, on ajoute 2 gouttes d'ammoniaque à 20%, 2 gouttes de solution de chlorure ferrique et verse goutte à goutte une solution d'eau oxygénée à 110 volumes (4,4g) jusqu'à décoloration du milieu réactionnel. On concentre sous pression réduite et l'huile obtenue est dissoute dans 200$cm^3$ d'éthanol absolu.

La solution est clarifiée par filtration puis concentrée sous pression réduite jusqu'à début de cristallisation. On refroidit à + 5°C, essore les cristaux blancs, lave par le minimum d'alcool glacé et sèche sous vide à 60-70°C. On obtient ainsi 13,3g de disulfure attendu sous la forme d'un solide blanc de point de fusion : 104°C.

Le spectre RMN'H 250 MHz est conforme à la structure attendue.

EXEMPLE IX

Préparation du bis[N-(hydroxy-2 éthyloxyéthyl butyramide)] disulfure

A une solution de 15g (0,072 mole) de N-(hydroxy-2 éthyloxyéthyl) mercapto-4 butyramide dans 75 $cm^3$ d'eau, agitée à température ambiante, on ajoute 2 gouttes d'ammoniaque à 20%, 2 gouttes de solution aqueuse de chlorure ferrique puis verse, goutte à goutte, une solution d'eau oxygénée à 110 volumes (4,05g) jusqu'à décoloration du milieu réactionnel.

On concentre ensuite sous pression réduite et on obtient une huile qui cristallise lentement. On dissout l'huile dans l'alcool absolu, clarifie par filtration et évapore à sec sous pression réduite. Après séchage prolongé sous vide à 30°C, on obtient 10,9g de disulfure attendu sous la forme d'un solide blanc de point de fusion : 43-45°C.

Le spectre RMN'H 250 MHz est conforme à la structure attendue.

EXEMPLE X

Préparation du bis[N- (butyryl-1) N'-(hydroxy-2 éthyl) pipérazine] disulfure.

A une solution de 10g (0,043mole) de N-(mercapto-4 butyryl-1) N'-(hydroxy-2 éthyl) pipérazine dans 70 $cm^3$ d'eau, agitée à température ambiante, on ajoute 2 gouttes d'ammoniaque à 20%, 2 gouttes de solution de chlorure ferrique puis verse goutte à goutte une solution d'eau oxygénée à 110 volumes (2,2g) jusqu'à décoloration du milieu réactionnel.

On clarifie par filtration, évapore à sec sous pression réduite à température inférieure à 40°C et sèche sous vide de façon prolongée à 40-50°C. On obtient ainsi 10,9g de disulfure attendu sous la forme d'une huile ambrée.

Le spectre RMN'H 80 MHz est conforme à la structure attendue.

EXEMPLES DE COMPOSITIONS

EXEMPLE 1

On prépare, selon l'invention, une composition réductrice de déformation permanente des cheveux en procédant au mélange des ingrédients suivants:

| | |
|---|---|
| - N-(mercapto-4 butyryl) N-méthylglucamine..... | 18g |
| - Monoéthanolamine .....qsp................ pH = | 8,5 |
| - Parfum....................................... | 0,1g |
| - Conservateur................................. | 0,5g |
| - Eau déminéralisée .....qsp................... | 100g |

Cette composition est appliquée sur des cheveux mouillés préalablement enroulés sur des rouleaux de mise en plis. Après avoir laissé agir la composition pendant environ 15min on rince abondamment à l'eau puis on applique la composition oxydante suivante:

| | |
|---|---|
| - Eau oxygénée .....qsp...................... | 8 volumes |
| - Stabilisant................................ | 0,3g |
| - Parfum..................................... | 0,1g |
| - Acide lactique .....qsp.................. pH = | 3 |
| - Eau déminéralisée ....qsp.................. | 100g |

On laisse agir la composition oxydante pendant environ 10min puis on enlève les rouleaux et rince abondamment la chevelure à l'eau.

Après séchage sous casque les cheveux présentent de belles boucles.

EXEMPLES 2 à 5

Selon le même mode d'application que décrit à l'exemple 1 ci-dessus on a réalisé la déformation permanente des cheveux à l'aide des compositions réductrices et oxydantes suivantes:

EXEMPLE 2

Composition réductrice

- N-(hydroxy-2 éthyloxyéthyl) mercapto-4 butyramide.................................. 12g
- Monoéthanolamine ....qsp..................pH = 9
- Parfum........................................ 0,05g
- Conservateur................................ 0,3 g
- Eau déminéralisée ....qsp.................... 100g

Composition oxydante

- $H_2O_2$ qsp.................. 8 volumes
- Stabilisants................................ 0,4g
- Parfum ..................................... 0,1g
- Acide lactique ....qsp ...................pH = 3
- Eau déminéralisée ...qsp. .................. 100g

EXEMPLE 3

Composition réductrice

- N-(dihydroxy-2,3 propyl) mercapto-4 butyramide................................. 10g
- Monoéthanolamine ....qsp................. pH = 8,5
- Parfum....................................... 0,1g
- Conservateur ................................ 0,2g
- Eau déminéralisée ....qsp .................. 100g

Composition oxydante

- $H_2O_2$ ....qsp................ 8 volumes
- Stabilisants................................ 0,3g
- Parfum ..................................... 0,1g
- Acide lactique ....qsp ...................pH = 3
- Eau déminéralisée ....qsp.................... 100g

EXEMPLE 4

Composition réductrice

- N-(hydroxy-2 éthyl) mercapto-4 butyramide.... 7g
- Ammoniaque ....qsp........................pH = 8,5
- Parfum................................... 0,2
- Conservateur ............................ 0,3
- Eau déminéralisée ....qsp ................. 100g

Composition oxydante

- $H_2O_2$ qsp.................... 8 volumes
- Stabilisants............................ 0,3
- Parfum .................................. 0,05
- Acide lactique qsp ..................... pH = 3
- Eau déminéralisée qsp .................... 100g

EXEMPLE 5

Composition réductrice

- N-(mercapto-4 butyryl-1) N'-(hydroxy-2 éthyl) pipérazine................................ 15g
- Ammoniaque ....qsp........................pH = 9
- Parfum................................... 0,1
- Conservateur ............................ 0,2
- Eau déminéralisée ....qsp ................. 100g

Composition oxydante

- $H_2O_2$ ....qsp................. 8 volumes
- Stabilisants............................ 0,3
- Parfum .................................. 0,1
- Acide lactique ....qsp ..................pH = 3
- Eau déminéralisée ....qsp ................. 100g

EXEMPLE 6

On prépare selon l'invention, une composition réductrice de déformation permanente des cheveux en procédant au mélange des ingrédients suivants :

- N-(hydroxy-2 éthyl) mercapto-4 butyramide ................................ 6g
- Acide thioglycolique ..................... 5g
- Stabilisant ............................. 0,2g

- Chlorure d'Oléocétyl diméthyl ammonium ...... 1,5g
- Ammoniaque ....qs ........................pH = 8,4
- Parfum ...................................... 0,1g
- Eau déminéralisée .........qsp............... 100g

On applique cette composition sur des cheveux mouillés, préalablement enroulés sur des bigoudis. On laisse agir 15 minutes puis on rince abondamment à l'eau. On applique alors, la composition oxydante suivante :

- Eau oxygénée ....qsp...................... 8 volumes
- Stabilisant ............................... 0,2g
- Alcool oléique à 20 moles
  d'oxyde d'éthylène ........................ 1,5g (MA)
- Acide citrique ..........qsp..............pH = 3
- Eau déminéralisée qsp ..................... 100g

On laisse agir la composition oxydante pendant 10 minutes. On rince à l'eau puis on enlève les bigoudis.

Après séchage sous casque, on constate que les cheveux présentent de belles boucles avec un bon degré de frisure.

EXEMPLES 7 à 10

Selon le même mode opératoire que celui décrit à l'exemple 6 ci-dessus, on a réalisé l'ondulation permanente des cheveux à l'aide des compositions réductrices et oxydantes suivantes :

EXEMPLE 7

Composition réductrice:

- N-(dihydroxy-2,3 propyl) mercapto-4 butyramide 8g
- Chlorhydrate de cystéamine ................. 4g
- Amido propyl bétaïne d'acide gras de coprah 2g
- Stabilisant ............................... 0,25g
- Ammoniaque ....qsp........................pH = 8,6
- Parfum .................................... 0,3g
- Eau déminéralisée ....qsp .................. 100g

Composition oxydante:

- Bromate de sodium ......................... 8g
- Triéthanolamine ....qsp ..................pH = 7,5
- Phosphate monosodique, monohydraté ......... 0,3g
- Phosphate trisodique ...................... 0,5g
- Parfum ....qs
- Eau déminéralisée ....qsp ................. 100g

EXEMPLE 8

Composition réductrice :

- N-(hydroxy-2 éthyl) mercapto-4 butyramide ... 6g
- Cystéïne .................................. 4,5g
- Lauryl éther sulfate d'ammonium ............ 1,5g
- Monoéthanolamine ....qsp..................pH = 8,8
- Parfum ......qs
- Eau déminéralisée ....qsp.................. 100g

Composition oxydante :

- Eau oxygénée à 200 volumes ................ 4,8g
- Stabilisants : Sulfate d'hydroxy-8 quinoléïne et phénacétine ........................... 0,06g
- Acide citrique ......qsp..................pH = 3
- Eau déminéralisée ...qsp .................. 100g

EXEMPLE 9

Composition réductrice :

- N-(dihydroxy-2,3 propyl) mercapto-4 butyramide 9g
- bis[N-(dihydroxy-2,3 propyl butyramide)] disulfure ................................ 5g
- Chlorure d'oléocétyl diméthyl ammonium ..... 1,7g
- Stabilisant ............................... 0,2g
- Ammoniaque ......qsp....................pH = 8,6
- Parfum ........qs
- Eau déminéralisée ............qsp........... 100g

Composition oxydante :

- Eau oxygénée à 200 vol .................... 4,8g
- Stabilisants : sulfate d'hydroxy-8 quinoléïne et phénacétine .................. 0,06g
- Lauryl éther sulfate d'ammonium ............ 2 g
- Acide citrique ....qsp..................pH = 3,5
- Eau déminéralisée ..........qsp ............ 100g

EXEMPLE 10

Composition réductrice

- N-(hydroxy-2 éthyl) mercapto-4 butyramide ................................ 6 g
- Acide thioglycolique ..................... 5 g
- Stabilisant ............................... 0,2 g
- Chlorure d'oléocétyl diméthyl ammonium .... 1,5 g
- Ammoniaque ....qsp........................pH = 8,4
- Parfum .......qs
- Eau déminéralisée .......... qsp .......... 100g

Composition oxydante

- Eau oxygénée à 200 volumes ................ 2 g
- Stabilisants .............................. 0,06g
- Chlorure d'oléocétyl diméthylammonium ..... 1,7 g
- Acide citrique ....qsp.....................pH = 3,0
- Eau déminéralisée ..........qsp............. 100g

**Revendications**

1. Mercapto-4 butyramides N-mono ou N,N-disubstitués par un radical mono ou polyhydroxyalkyle, caractérisés par le fait qu'ils correspondent à la formule générale suivante:

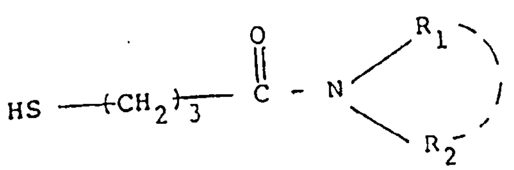

(I)

dans laquelle:

$R_1$ représente un atome d'hydrogène, un radical alkyle inférieur ou un radical monohydroxyalkyle ou polyhydroxyalkyle ayant de 1 à 6 atomes de carbone, éventuellement interrompus par un atome d'oxygène, d'azote ou de soufre,

et $R_2$ représente un radical monohydroxyalkyle ou polyhydroxyalkyle ayant de 1 à 6 atomes de carbone éventuellement interrompus par un atome d'oxygène, d'azote ou de soufre,

ou $R_1$ et $R_2$ , pris ensemble avec l'atome d'azote, forment un cycle pipérazine substitué en 4 par un radical mono ou polyhydroxyalkyle ayant de 1 à 6 atomes de carbone, et leurs disulfures correspondants.

2. Composés selon la revendication 1, caractérisés par le fait que le radical alkyle inférieur est le radical méthyle, éthyle, propyle, isopropyle ou butyle.

3. Composés selon la revendication 1, caractérisés par le fait que le radical monohydroxyalkyle ou polyhydroxyalkyle ayant de 1 à 6 atomes de carbone, éventuellement interrompus par un atome d'oxygène, d'azote ou de soufre est le radical hydroxy-2 éthyle, dihydroxy-2,3 propyle, diméthyl-1,1 hydroxy-2 éthyle,

pentahydroxy-2,3,4,5,6 hexyle et hydroxy-2 éthyloxyéthyle.

4. Composés selon l'une quelconque des revendications 1 à 3, caractérisés par le fait qu'ils sont:
   - le N-(hydroxy-2 éthyl) mercapto-4 butyramide,
   - le N-(hydroxy-2 éthyloxyéthyl) mercapto-4 butyramide,
   - le N-(dihydroxy-2,3 propyl) mercapto-4 butyramide,
   - le N,N-di(hydroxy-2 éthyl) mercapto-4 butyramide,
   - la N-(mercapto-4 butyryl) N-méthylglucamine,
   - la N-(mercapto-4 butyryl-1) N'-(hydroxy-2 éthyl) pipérazine, et
   - le N-(diméthyl-1,1 hydroxy-2 éthyl) mercapto-4 butyramide.

5. Procédé de préparation des mercapto-4 butyramides selon les revendications 1 à 4, caractérisé par le fait qu'il consiste à faire réagir une amine de formule :

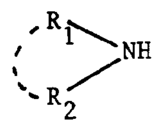

$R_1$ et $R_2$ ayant les mêmes significations qu'à la revendication 1, sur la γ-thiobutyrolactone, la réaction étant conduite, avec ou sans solvant, à une température comprise entre 50 et 110°C.

6. Procédé selon la revendication 5, caractérisé par le fait que la réaction est conduite en présence de toluène ou d'un alcool pris dans le groupe constitué par le méthanol, l'éthanol, l'isopropanol ou le butanol.

7. Procédé selon l'une quelconque des revendications 5 et 6, caractérisé par le fait que l'amine est l'éthanolamine, la diéthanolamine, la diglycolamine, la méglumine, la dihydroxy-2,3 propylamine, l'amino-2 méthyl-2 propanol de l'hydroxy-2 éthyl pipérazine.

8. Composés selon la revendication 1 sous forme de leurs disulfures ayant la formule générale suivante :

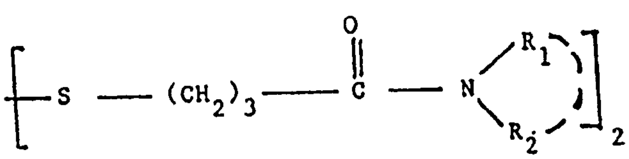

(II)

dans laquelle :
$R_1$ et $R_2$ sont tels que définis à la revendication 1.

9. Composés selon la revendication 8 caractérisés par le fait qu'ils sont :
   le bis[N-(dihydroxy2,3 propyl butyramide)]disulfure),
   le bis[N-(hydroxy-2 éthyloxyéthyl butyramide)]disulfure, et
   le bis [N-(butyryl-1) N'-(hydroxy-2 éthyl) pipérazine]disulfure.

10. Composition cosmétique réductrice pour le premier temps d'une opération de déformation permanente des cheveux, caractérisée par le fait qu'elle contient, dans un véhicule cosmétique approprié, en tant qu'agent réducteur, au moins un mercapto-4 butyramide de formule (I) selon l'une quelconque des revendications 1 à 4, ou obtenu selon l'une quelconque des revendications 5 à 7.

11. Composition selon la revendication 10, caractérisée par le fait que l'agent réducteur est présent à une concentration comprise entre 2 et 20% en poids et de préférence entre 5 et 10% en poids par rapport au poids total de la composition.

12. Composition selon l'une quelconque des revendications 10 et 11, caractérisée par le fait que son pH est compris entre 4 et 11.

13. Composition selon l'une quelconque des revendications 10 à 12 caractérisée par le fait qu'elle contient un disulfure selon l'une quelconque des revendications 8 et 9.

14. Composition selon l'une quelconque des revendications 10 à 13, caractérisée par le fait qu'elle contient en outre au moins un polymère cationique, un agent adoucissant, un hydrolysat de protéine, une cire, un agent opacifiant, un parfum, un colorant, un tensio-actif non-ionique ou cationique ou un agent traitant.

15. Procédé de déformation permanente des cheveux consistant dans une première étape à réduire les liaisons disulfures de la kératine par application d'une composition réductrice puis dans une seconde étape à reformer lesdites liaisons par application d'une composition oxydante, caractérisé par le fait que l'étape de réduction est réalisée à l'aide d'une composition cosmétique réductrice telle que revendiquée selon l'une quelconque des revendications 10 à 14.

16. Procédé selon la revendication 15, caractérisé par le fait que l'on laisse agir la composition réductrice pendant un temps compris entre 5 et 60 min.

## Patentansprüche

1. Durch einen Mono- oder Polyhydroxyalkylrest N-monosubstituierte oder N, N-disubstitutierte 4-Mercaptobutyramide, dadurch gekennzeichnet, daß sie der folgenden allgemeinen Formel entsprechen:

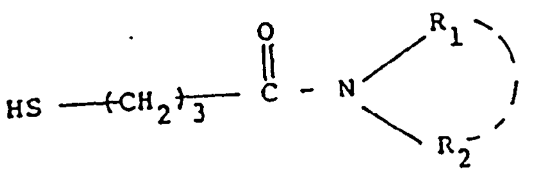

(I)

worin

$R_1$ für ein wasserstoffatom, einen niederen Alkylrest oder einen Monohydroxyalkylrest oder Polyhydroxyalkylrest steht, der 1 bis 6 Kohlenstoffatome aufweist und gegebenenfalls durch ein Sauerstoff-, Stickstoff- oder Schwefelatom unterbrochen ist, und

$R_2$ für einen Monohydroxyalkylrest oder Polyhydroxyalkylrest steht, der 1 bis 6 Kohlenstoffatome aufweist und gegebenenfalls durch ein Sauerstoff-, Stickstoff- oder Schwefelatom unterbrochen ist, oder

$R_1$ und $R_2$, mit dem Stickstoffatom zusammengenommen, einen Piperazinring bilden, der in 4-Stellung durch einen Monohydroxyalkylrest oder Polyhydroxyalkylrest mit 1 bis 6 Kohlenstoffatomen substituiert ist,

und die entsprechenden Disulfide.

2. Verbindungen nach Anspruch 1, daß der Niederalkylrest ein Methylrest, Ethylrest, Propylrest, Isopropylrest oder Butylrest ist.

3. Verbindungen nach Anspruch 1, dadurch gekennzeichnet, daß der Monohydroxyalkylrest oder Polyhydroxyalkylrest, der 1 bis 6 Kohlenstoffatome aufweist und gegebenenfalls durch ein Sauerstoff-, Stickstoff- oder Schwefelatom unterbrochen ist, ein 2-Hydroxyethylrest, 2,3-Dihydroxypropylrest, 1, 1-Dimethyl-2-hydroxyethylrest, 2,3,4,5,6-Pentahydroxyhexylrest und 2-Hydroxyethyloxyethylrest ist.

4. Verbindungen nach irgendeinem der Ansprüche 1 bis 3, dadurch gekennzeichnet, daß es die folgenden Verbindungen sind:
   - N-(2-hydroxyethyl-)4-mercaptobutyramid;
   - N-(2-hydroxyethyloxyethyl-)4-mercaptobutyramid;
   - N-(2,3-dihydroxypropyl-)4-mercaptobutyramid;
   - N,N-Bis(2-hydroxyethyl-)4-mercaptobutyramid;
   - N-(4-mercaptobutyryl)N-methylglucamin;
   - N-(4-mercapto- 1-butyryl-)N'-(2-hydroxyethyl-)piperazin;
   
   und
   - N-(1,1-dimethyl-2-hydroxyethyl-)4-mercaptobutyramid.

5. Verfahren zur Herstellung von 4-Mercaptobutyramiden gemäß Patentansprüchen 1 bis 4, dadurch gekennzeichnet, daß es aus einer Umsetzung eines Amins der Formel

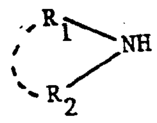

worin

$R_1$ und $R_2$ die gleichen Bedeutungen wie in Patentanspruch 1 aufweisen, mit $\gamma$-Thiobutyrolacton besteht, wobei die Reaktion mit oder ohne Lösungsmittel bei einer Temperatur im Bereich von 50 bis 110°C durchgeführt wird.

6. Verfahren nach Anspruch 5, dadurch gekennzeichnet, daß die Reaktion in Gegenwart von Toluol oder von einem Alkohol aus der aus Methanol, Ethanol, Isopropanol und Butanol bestehenden Gruppe durchgeführt wird.

7. Verfahren nach irgendeinem der Ansprüche 5 und 6, dadurch gekennzeichnet, daß das Amin Ethanolamin, Diethanolamin, Diglycolamin, Meglumin, 2,3-Dihydroxypropylamin, 2-Amino-2-methylpropanol oder 2-Hydroxyethylpiperazin ist.

8. Verbindungen nach Anspruch 1 in Form ihrer Disulfide, welche die folgende allgemeine Formel aufweisen:

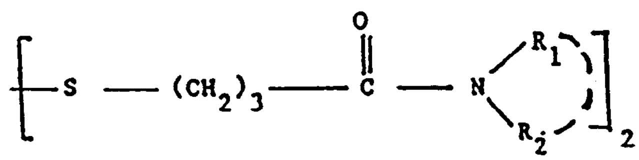

worin

$R_1$ und $R_2$ die in Patentanspruch 1 definierte Bedeutung aufweisen.

9. Verbindungen nach Anspruch 8, dadurch gekennzeichnet, daß es die folgenden Verbindungen sind:
– Bis-[N-(2,3-dihydroxypropylbutyramid-)]disulfid;
– Bis-[N-(2-hydroxyethyloxyethylbutyramid-)]disulfid;
und
– Bis-[N-(1-butyryl)N'-(2-hydroxyethyl-)piperazin]disulfid.

10. In der ersten Zeit eines Verfahrens zur dauernden Verformung der Haare reduzierend wirkende kosmetische Zubereitung, dadurch gekennzeichnet, daß sie in einem geeigneten kosmetischen Träger als Reduktionsmittel wenigstens ein 4-Mercaptobutyramid der Formel (I) nach irgendeinem der Ansprüche 1 bis 4 oder erhalten nach irgendeinem der Ansprüche 5 bis 7 enthält.

11. Zubereitung nach Anspruch 10, dadurch gekennzeichnet, daß das Reduktionsmittel in einer Konzentration im Bereich von 2 bis 20 Gew. - % und vorzugsweise im Bereich von 5 bis 10 Gew.-%, bezogen auf das gesamte Gewicht der Zubereitung, zugegen ist.

12. Zubereitung nach irgendeinem der Ansprüche 10 und 11, dadurch gekennzeichnet, daß ihr pH-Wert zwischen 4 und 11 liegt.

13. Zubereitung nach irgendeinem der Ansprüche 10 bis 12, dadurch gekennzeichnet, daß sie ein Disulfid nach irgendeinem der Ansprüche 8 und 9 enthält.

14. Zubereitung nach irgendeinem der Ansprüche 10 bis 13, dadurch gekennzeichnet, daß sie darüber hinaus wenigstens ein kationisches Polymer, ein weichmachendes Mittel, ein Protein-Hydrolysat, ein wachs, ein opazifizierendes Mittel, ein Parfum, ein Färbemittel, ein nichtionisches oder kationisches oberflächenaktives Mittel oder ein Behandlungsmittel enthält.

**15.** Verfahren zur dauerhaften Verformung der Haare, welches in einem ersten Schritt aus einer Reduktion der Disulfid-Bindungen des Keratins durch Aufbringung einer reduzierenden Zubereitung und danach in einem zweiten Schritt aus einer Neubildung der genannten Bindungen durch Anwendung einer oxidierenden Zubereitung besteht, dadurch gekennzeichnet, daß der Reduktionsschritt mit Hilfe einer reduzierenden kosmetischen Zubereitung durchgeführt wird, wie sie in irgendeinem der Ansprüche 10 bis 14 beansprucht ist.

**16.** Verfahren nach Anspruch 15, dadurch gekennzeichnet, daß man die reduzierende Zubereitung während einer Zeitdauer im Bereich von 5 bis 60 Minuten einwirken läßt.

## Claims

**1.** 4-Mercaptobutyramides which are N-mono- or N,N-disubstituted by a mono- or polyhydroxyalkyl radical, characterised in that they correspond to the following general formula:

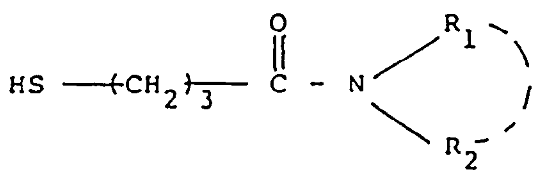

(I)

in which:

$R_1$ represents a hydrogen atom, a lower alkyl radical or a monohydroxyalkyl or polyhydroxyalkyl radical having 1 to 6 carbon atoms, optionally interrupted by an oxygen, nitrogen or sulphur atom,

and $R_2$ represents a monohydroxyalkyl or polyhydroxyalkyl radical having 1 to 6 carbon atoms, optionally interrupted by an oxygen, nitrogen or sulphur atom,

or $R_1$ and $R_2$, taken together with the nitrogen atom, form a piperazine ring substituted in position 4 by a mono- or polyhydroxyalkyl radical having 1 to 6 carbon atoms, and their corresponding disulphides.

**2.** Compounds according to Claim 1, characterised in that the lower alkyl radical is a methyl, ethyl, propyl, isopropyl or butyl radical.

**3.** Compounds according to Claim 1, characterised in that the monohydroxyalkyl or polyhydroxyalkyl radical having 1 to 6 carbon atoms, optionally interrupted by an oxygen, nitrogen or sulphur atom is a 2-hydroxyethyl, 2,3-dihydroxypropyl, 1,1-dimethyl-2-hydroxyethyl, 2,3,4,5,6-pentahydroxyhexyl or 2-hydroxyethyloxyethyl radical.

**4.** Compounds according to any one of Claims 1 to 3, characterised in that they are:
- N-(2-hydroxyethyl)-4-mercaptobutyramide,
- N-(2-hydroxyethyloxyethyl)-4-mercaptobutyramide,
- N-(2,3-dihydroxypropyl)-4-mercaptobutyramide,
- N,N-di(2-hydroxyethyl)-4-mercaptobutyramide,
- N-(4-mercaptobutyryl)-N-methylglucamine,
- N-(4-mercapto-1-butyryl)-N′-(2-hydroxyethyl)-piperazine, and
- N-(1,1-dimethyl-2-hydroxyethyl)-4-mercaptobutyramide.

**5.** Process for preparing the 4-mercaptobutyramides according to Claims 1 to 4, characterised in that it consists in reacting an amine of formula:

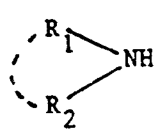

$R_1$ and $R_2$ having the same meanings as in Claim 1, with γ-thiobutyrolactone,
the reaction being carried out, with or without solvent, at a temperature of between 50 and 110°C.

6. Process according to Claim 5, characterised in that the reaction is carried out in the presence of toluene or an alcohol chosen from the group consisting of methanol, ethanol, isopropanol and butanol.

7. Process according to either of Claims 5 and 6, characterised in that the amine is ethanolamine, diethanolamine, diglycolamine, meglumine, 2, 3-dihydroxypropylamine, 2-amino-2 -methylpropanol or 2-hydroxyethylpiperazine.

8. Compounds according to Claim 1, in the form of their disulphides of the following general formula:

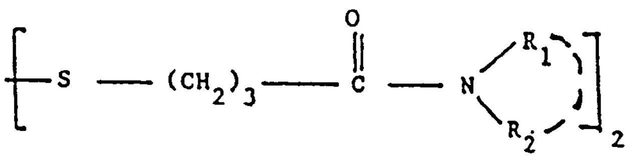

(II)

in which:
$R_1$ and $R_2$ are as defined in Claim 1.

9. Compounds according to Claim 8, characterised in that they are:
bis[N-(2,3-dihydroxylpropylbutyramide)]disulphide,
bis[N-(2-hydroxyethyloxyethylbutyramide)] disulphide, and
bis[N-(1-butyryl)-N′-(2-hydroxyethyl)piperazine] disulphide.

10. Reducing cosmetic composition for the first stage of an operation for the permanent deformation of the hair, characterised in that it contains, in an appropriate cosmetic vehicle, as reducing agent, at least one 4-mercaptobutyramide of formula (I) according to any one of Claims 1 to 4, or obtained according to any one of Claims 5 to 7.

11. Composition according to Claim 10, characterised in that the reducing agent is present at a concentration of between 2 and 20% by weight, and preferably between 5 and 10% by weight, relative to the total weight of the composition.

12. Composition according to either of Claims 10 and 11, characterised in that its pH is between 4 and 11.

13. Composition according to any one of Claims 10 to 12, characterised in that it contains a disulphide according to either of Claims 8 and 9.

14. Composition according to any one of Claims 10 to 13, characterised in that it contains, in addition, at least one cationic polymer, one demulcent, one protein hydrolysate, one wax, one opacifying agent, one perfume, one colorant, one nonionic or cationic surface-active agent or one treatment agent.

15. Process for the permanent deformation of the hair consisting, in a first stage, in reducing the disulphide bonds of keratin by applying a reducing composition and then, in a second stage, in reforming the said bonds by applying an oxidising composition, characterised in that the reducing stage is carried out by means of a reducing cosmetic composition as claimed in any one of Claims 10 to 14.

16. Process according to Claim 15, characterised in that the reducing composition is allowed to act for a period of between 5 and 60 min.